# EUROPEAN PATENT SPECIFICATION

(11) **EP 2 978 466 B1**
(45) Date of publication and mention of the grant of the patent: **18.04.2018**
(21) Application number: 14715101.3
(22) Date of filing: 28.03.2014
(51) Int. Cl.: A61M 1/10

(54) **BLOOD PUMP**
BLUTPUMPE
POMPE A SANG

(30) Priority: 28.03.2013 GB 201305758
(43) Date of publication of application: 03.02.2016
(73) Proprietor: Quanta Dialysis Technologies Limited, Alcester Warwickshire B49 6EU (GB)
(72) Inventor: BUCKBERRY, Clive, Warwick Warwickshire CV34 6AH (GB)
(74) Representative: Croston, David
(86) International application number: PCT/GB2014/051007
(87) International publication number: WO 2014/155137

(56) References cited:
- EP-A1- 1 362 604
- WO-A1-03/101510
- WO-A2-2005/089832
- WO-A2-2011/027117
- DE-A1- 19 837 667
- US-A- 4 923 444
- US-A1- 2009 137 940

## Description

The present invention relates to extracorporeal blood pumps, in particular, but not exclusively, to low haemolysis extracorporeal blood pumps, eg. see WO03/101510.

Blood pumps for the extracorporeal circulation of blood are used in a number of medical applications, for example in hemodialysis. Hemodialysis machines are large expensive machines which a patient typically attends a medical facility to use. Due to the risk of cross contamination of one patients blood with another it is desirable to dispose of blood pumps after just one use.

Some hemodialysis machines are suitable for use by a patient at home. In such machines it is still desirable to dispose of the blood pump after a single use to prevent clotted blood from a previous dialysis session re-entering the patient's body.

Conventionally, blood pumps are peristaltic pumps which are considered to be too expensive to dispose of after a single use.

Use of a membrane pump as a blood pump has provided a relatively cost effective means of providing single use blood pumps. Until now, such blood pumps have comprised a multi-part moulding, usually a pump part and a sensing part. Each part of a moulding carries a risk of errors in the moulding process which could have a negative effect on a patients blood being pumped through the blood pump. For example, moulding errors at flow ports in the blood pump can lead to stagnation of blood and ultimately clotting of the blood.

It is the purpose of the present invention to mitigate the above problems and to produce a blood pump that comprises a single moulding.

An aspect of the invention provides a blood pump comprising a cartridge, the cartridge comprising a first recess therein, said first recess having a surface, and a flexible diaphragm closing said first recess, the first recess and the flexible diaphragm defining a first pump chamber, said first pump chamber having an inlet and an outlet wherein the flexible diaphragm of the first pump chamber is movable between a first position, separated in use from the surface of the first recess, wherein in said first position said first pump chamber has a maximum volume, and a second position, substantially adjacent to the surface of the first recess, wherein in said second position said first pump chamber has a minimum volume, a pump driver arranged to interface with the cartridge, said pump driver operable to move the flexible diaphragm of the first pump chamber in a first direction into said first recess to, in use, pump blood from the chamber and to move the flexible diaphragm of the first pump chamber in a second direction away from the first recess to, in use, draw blood into said first pump chamber, wherein, the cartridge further comprises one or more sensor cavities defined by respective recesses in the cartridge, the, or each, recess being closed by a flexible diaphragm.

Providing a cartridge housing both a pump chamber for pumping a patients blood to/from a dialyser and a sensor cavity for measuring the pressure of blood pumped results in reduced manufacturing costs and fewer errors in manufacturing due to fewer components being produced.

In one embodiment, the cartridge further comprises a second recess therein, said second recess having a surface, and a flexible diaphragm closing said second recess, the second recess and the flexible diaphragm defining a second pump chamber, said second pump chamber having an inlet and an outlet wherein the flexible diaphragm of the second pump chamber is movable between a first position, separated in use from the surface of the second recess, in which said second pump chamber has a maximum volume, and a second position, substantially adjacent to the surface of the second recess, in which said second pump chamber has a minimum volume.

Provision of two pump chambers on the blood pump cartridge permits the blood pump to be used with single or twin needle configurations. A twin needle configuration is advantageous if greater flow of blood from the patient through the dialyser is required. Use of a twin needle configuration effectively allows for double the blood flow from the patient than use of a single needle configuration.

In one embodiment, the blood pump further comprises a platen, the platen having one or more recesses therein, each recess having a surface, the one or more recesses corresponding substantially in geometry to a recess in the cartridge and being separated therefrom by a flexible diaphragm associated therewith.

Advantageously, the surface(s) associated with the cartridge and the surface(s) associated with the platen provide a positive stop for the flexible diaphragm(s) thus defining the minimum and maximum volumes of the blood pump.

In one embodiment, the flexible diaphragm is pneumatically actuated between said first and second position.

In one embodiment, the flexible diaphragm, when in a first position contacts the surface of the first recess in the cartridge and, when in a second position contacts the surface of the corresponding recess in the platen.

In one embodiment, the first and second pump chambers can be operated in phase with one another.

Operating the first and second pump chambers in phase with one another allows use of the twin needle configuration introduced above.

In another embodiment, the first and second pump chambers can be operated out of phase with one another.

Operating the first and second pump chambers out of phase with one another allows the first pump chamber, for example, to be used to draw blood from the patients artery and to pump blood to the dialyser and allows the second pump chamber, for example, to draw treated blood from the dialyser and return it to the patients vein.

In one embodiment, the blood pump further comprises an inlet valve to the first pump chamber and an inlet valve to the second pump chamber, each of said inlet valves being disposed in an inlet channel.

In one embodiment, the blood pump further comprises an outlet valve from the first pump chamber and an outlet valve from the second pump chamber, each outlet valve being disposed in an outlet channel.

The provision of inlet valves to and outlet valves from each pump chamber permits the first and second pump chambers to be used together, in isolation, in phase with each other or out of phase with each other. Such a configuration permits the blood pump to be used for either single needle or twin needle extracorporeal blood treatment.

In one embodiment, the, or each sensor cavity is hemispherical.

In one embodiment, the, or each sensor cavity further comprises a surface, said surface defining a flat bottom in a recess. Such an arrangement facilitates the reflection of an ultrasound signal or optical signal, enhancing detection fidelity.

An ultrasonic transducer is used to measure a characteristic of the patients blood as it passes through the sensor cavity. The ultrasonic transducer is provided external to the sensor cavity and is in contact with the flexible diaphragm closing the sensor cavity. Provision of a flat bottom in the sensor cavity, internal or external, aids reflection of the ultrasonic waves emitted by the ultrasonic transducer.

In one embodiment, the inlet and outlet of the, or each, sensor cavity are tangential. Provision of tangential inlets and outlets is desirable to reduce stasis of the patients blood within the sensor cavity. The tangential inlets and outlets do not have areas where blood could collect and not circulate and encourage the blood to swirl within the sensor cavity.

In one embodiment, the first pump chamber has a common inlet and outlet and the second pump chamber has a common inlet and outlet.

In one embodiment, the blood pump further comprises an arterial blood inlet, a dialyser blood outlet, a dialyser blood inlet and a venous blood outlet.

In one embodiment, the blood pump comprises three sensor cavities.

In one embodiment, the sensor cavities respectively define an arterial pressure chamber, a dialyser pressure chamber and a venous pressure chamber.

In one embodiment, the blood pump further comprises an inlet channel between the arterial blood inlet and the first pump chamber and the second pump chamber.

In one embodiment, the blood pump further comprises an outlet channel between the dialyser blood outlet and the first pump chamber and the second pump chamber.

In one embodiment, the blood pump further comprises an inlet valve to the first pump chamber and an inlet valve to the second pump chamber, each inlet valve being disposed in the inlet channel.

In one embodiment the arterial pressure chamber is provided downstream of the arterial blood inlet and upstream of the first and second pump chambers.

In one embodiment, the dialyser pressure chamber is provided downstream of the first and second pump chambers and upstream of the dialyser blood outlet.

In one embodiment, the venous pressure chamber is provided downstream of the dialyser blood inlet and upstream of the venous blood outlet.

In one embodiment, the, or each, pump chamber, sensor cavity, valve and channel is provided on a common datum face.

In one embodiment, the blood pump is disposable.

Provision of a disposable blood pump is desirable to remove the need for cleaning of the blood pump after each extracorporeal blood treatment session.

In one embodiment, the flexible diaphragm of the, or each, pump chamber and the, or each, sensor cavity is formed from a single sheet of material.

In one embodiment, the sheet of material defining respective diaphragms is vacuum attached to the cartridge in a region around the, or each, sensor cavity.

Vacuum attaching the flexible diaphragms of the sensor cavities to the cartridge increases reflection from an ultrasonic transducer through the sensor cavities and reduces reflection at the interface between the flexible diaphragm and the cartridge to optimise measurements taken by the transducer by discriminating the ultrasound transducer signal from background noise.

A further aspect of the invention provides a blood pump comprising a cartridge, the cartridge comprising one or more pump chambers and one or more sensor cavities, the, or each pump chamber and the, or each, sensor cavity being provided on a common datum face.

Advantageously, providing each of the features of the blood pump on a common datum face simplifies production of the cartridge and permits the cartridge to be manufactured from a single piece of material. This is desirable as a single cartridge is simpler to load and unload from a hemodialysis machine thus resulting in fewer user errors in using the machine.

Embodiments of the invention will now be described, by way of example only, with reference to the following drawings in which:
Figure 1a is a schematic plan of a blood pump according to an embodiment of the invention viewed from one side;
Figure 1b is a schematic plan of a blood pump according to an embodiment of the invention viewed from the other side;
Figure 2a is an enlarged view of the sensor cavity section of the blood pump of figure 1a in isolation (rotated anti-clockwise by 90°);
Figure 2b is a side view of the sensor cavity section of figure 2a;
Figure 2c is a cross-sectional view of the arterial pressure chamber taken at lines A-A of figure 2a;
Figure 2d is a cross-sectional view of the sensor cavity section mid-plane taken at lines B-B of figure 2b;
Figure 2e is a cross-sectional view of the sensor cavity section mid-plane taken at lines C-C of figure 2a;
Figure 2f is a cross-sectional view of an alternative sensor cavity section represented similarly as figure 2e; and
Figure 3 is a schematic side view of a blood pump according to an embodiment of the invention.

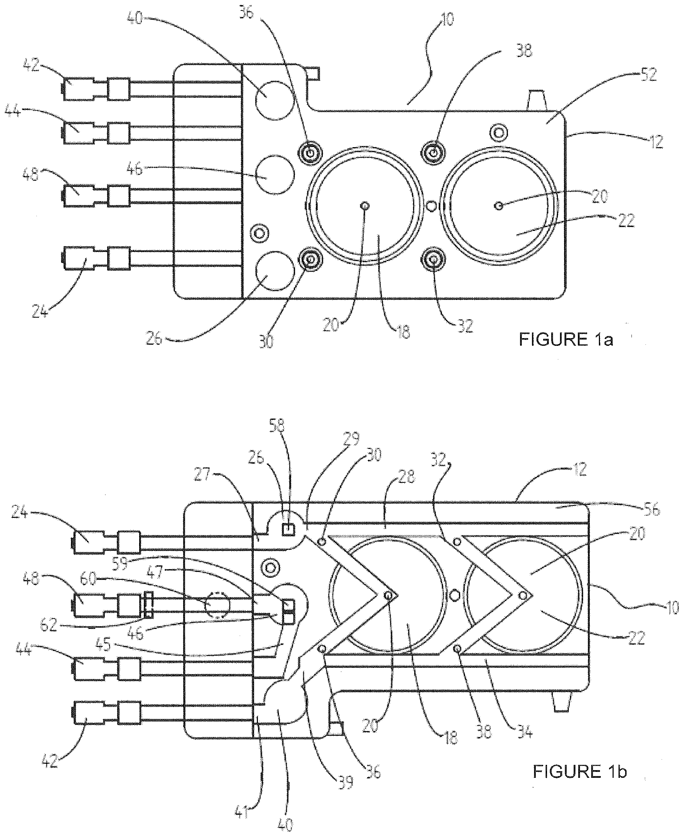

Referring to Figures 1 to 3, an embodiment of the invention provides a blood pump 10 comprising a pump cartridge 12 manufactured from a plastic shell and having a concave recessed surface 14 covered by a flexible diaphragm 16. The recessed surface 14 and the flexible diaphragm 16 define a pump chamber 18 of conical, concave or frustroconical shape and having at the apex thereof a common inlet and outlet 20 for both allowing blood to flow into the pump chamber 18 and to be pumped from the pump chamber 18.

In the illustrated example, the cartridge 12 provides two pump chambers 18, 22 but it will be appreciated that the number of pump chambers is not intended to be limited.

Blood is received via a needle from a patients artery through an arterial flow port 24 into a sensor cavity 26 defining an arterial pressure chamber. Blood enters the arterial pressure chamber 26 via an arterial pressure chamber inlet 27. The arterial pressure chamber 26 measures the pressure of blood from the patients artery and the output may be used to distinguish between a correctly placed needle and a dislodged needle. From the arterial pressure chamber 26 blood is received into an inlet channel 28, via an arterial pressure chamber outlet 29. The inlet channel 28 is provided with an inlet valve 30 to the first blood pump chamber 18 and an inlet valve 32 to the second blood pump chamber 22.

The first and second blood pump chambers 18, 22 are selectively operable to run in or out of phase with one another or in or out of phase with further pumps forming part of a hemodialysis machine. From the first and second blood pump chambers 18, 22, blood is pumped to an outlet channel 34, via an outlet valve 36 of the first blood pump chamber 18 and via an outlet valve 38 of the second blood pump chamber 22.

From the outlet channel 34 blood passes to a dialyser through a sensor cavity 40 defining a pre-dialyser pressure chamber. The pre-dialyser pressure chamber 40 has an inlet 39 and an outlet 41. Blood passes out of the cartridge 12 via a dialyser outlet port 42. The pre-dialyser pressure chamber 40 measures blood pressure prior to entering the dialyser to allow the flow rate of blood passing through the dialyser to be calculated.

After passing through the dialyser blood re-enters the cartridge 12 via a dialyser inlet port 44 into a sensor cavity 46 defining a venous pressure chamber. The venous pressure chamber 46 has an inlet 45 and an outlet 47. The venous pressure chamber 46 measures blood pressure blood prior to returning to the patients vein from the cartridge 12 via a venous outlet port 48. The venous pressure chamber 46 sends a signal to a modulated control valve 82 in response to variations in the venous return blood pressure caused by the patient moving around.

Each sensor cavity 26, 40, 46 comprises a concave recess covered by a flexible diaphragm to define the respective hemispherical pressure chambers. The hemispherical pressure chambers thereby present an upturned bowl shape, which minimizes the surface area to volume ratio, and precludes the provision of corners which would otherwise provide stagnation points for the flow. The inlets and outlets from each sensor cavity are arranged as will be described below, to further minimise stasis in the blood. Stasis occurs when flow of fluid is interrupted by an obstruction. Blood entering a sensor cavity through an inlet swirls within the sensor cavity until it exits through an outlet.

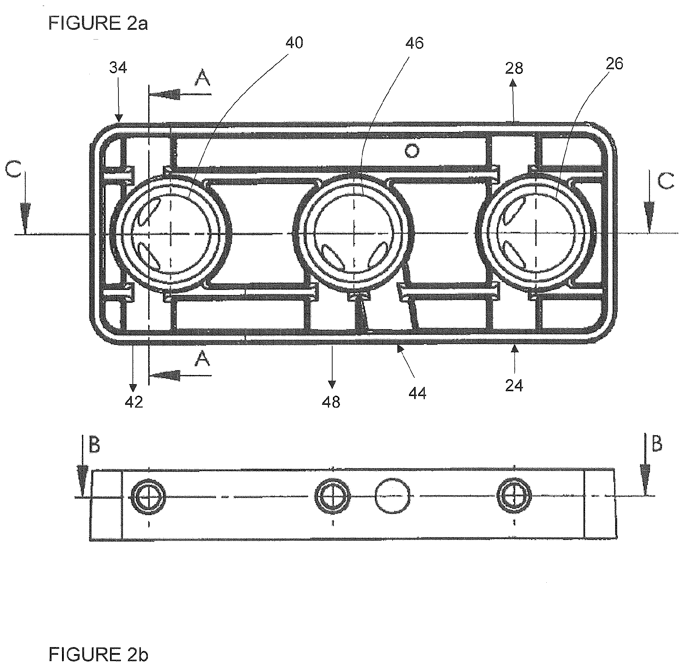

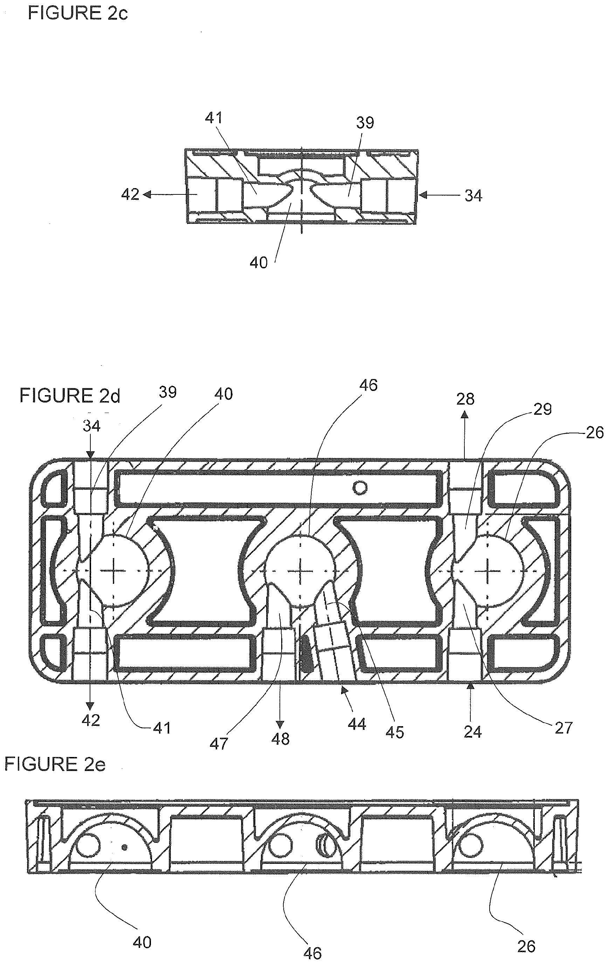

With reference to figures 2a to 2e, the sensor cavities 26, 40, 46 define respective hemispherical pressure chambers, bounded on one side by the flexible diaphragm. Each inlet enters the pressure chambers at an elliptical orifice, as the cylindrically shaped channels 24, 34, 44 meet the hemispherical pressure chamber wall. Thus their respective inlets 27, 39, 45 provide a shear profile across the inlet orifice in the blood flow as it enters the cavities which enhances mixing.

Furthermore, in the cases of the arterial pressure chamber 26 and pre-dialyser pressure chamber 40, the proximity of their respective inlets 27, 45 to the pressure chamber wall provides a surface drag, decelerating the blood flow asymmetrically, also enhancing mixing.

In the case of the venous pressure chamber 46, the inlet 45 and outlet 47 are misaligned, with the inlet 44 being non-tangential with the venous pressure chamber wall to prevent the blood flow from simply attaching to the wall and exiting the venous pressure chamber 46 at the outlet 47 following a "U-turn" flow path, forcing the blood flow to impinge upon the cavity wall opposite the inlet 45.

The unsteady flow pattern created by the arrangement of the cavities 26, 40, 46 and their respective inlet and outlets 27, 39, 45, 29, 41, 47, coupled with the pulsible nature of the membrane pump referred to above minimises stasis and maximises mixing of the blood in a spacially compact arrangement.

Although the illustrated embodiment is described having an arterial pressure chamber 26, venous pressure chamber 46 and pre-dialyser pressure chamber 40, the provided sensor cavities are not intended to be limited for such purposes. For example, the sensor cavities can be used for detecting pressure, bubbles, blood, hematocrit and urea clearance, for example by means of known apparatus and techniques. Additionally, although three sensor cavities are shown in the illustrated embodiment, it will be appreciated that more or less than three sensor cavities could be provided.

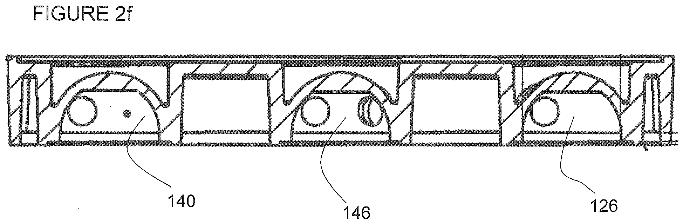

In an alternative sensor cavity (see Figure 2f) the hemispherical pressure chambers are flattened so as to define two parallel surfaces, one surface being the flexible diaphragm and the other surface being a flat bottom in the recess. Similar reference numbers are used to identify similar features, prefixed with a '1' to denote that those features are of the alternate sensor cavity.

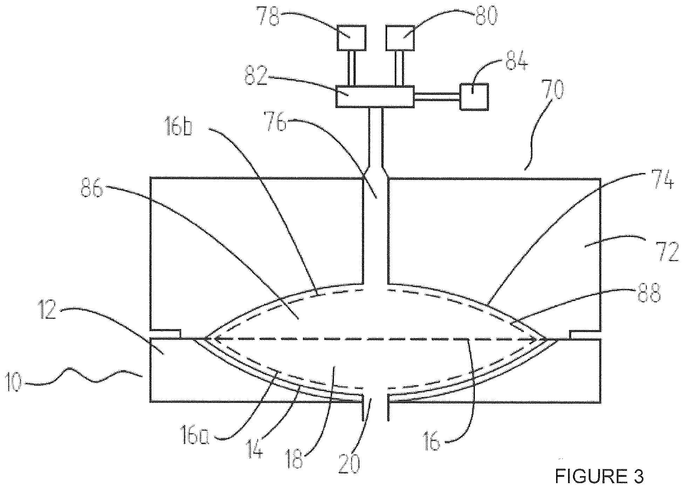

The arterial pressure chamber 46, of the illustrated embodiment, comprises a pressure transducer 58 for controlling a modulated valve 82 (see Figure 3) provided on a hemodialysis machine, as described below. The pressure transducer 58 provides feedback to a controller 84 to prevent excessive vacuum being used to draw blood into the pump chamber(s) 18, 22, as described below.

The pump chambers, sensor cavities and valves all share a common datum face 52 covered by a single, common flexible sheet of material defining the respective pump chamber and sensor cavity diaphragms. In the illustrated embodiment, the inlet and outlet channels 28, 34 are provided on the opposing face 56 to the common datum face 52 of the cartridge and are closed by a second single, common flexible sheet of material (not shown).

In an alternative embodiment the inlet and outlet channels 28, 34 are disposed between and sealed by both flexible sheets of material.

The flexible sheet of material sealing the common datum surface 52 is held against the cartridge 12 by vacuum around each sensor cavity. The flexible sheet of material sealing the common datum surface 52 is attached by adhesive to the cartridge 12 at its periphery and around each pump chamber and valve to define the respective diaphragms. The flexible sheet of material (not shown) sealing the surface 56 opposing the common datum surface 52 is attached to the cartridge 12 by adhesive.

The cartridge 12 contains a thrombus trap 59 and a bubble trap 60, of known types, moulded therein in the form of flow through cells. The thrombus trap 59 could be located, for example, in a sensor cavity. The bubble trap 60 comprises a blood inlet (not shown), a blood outlet (not shown) and a vent (not shown) to the common datum face 52 side of the cartridge 12.

A venous clamp 62 is attached to the venous return line to the patient. Upon activation of a hemodialysis machine safety system, the venous clamp 62 is applied to prevent further flow of blood to the patient. In use, the safety system is adapted to also deactivate the blood pump chambers 18, 22 to prevent further blood being pumped from the patient.

With reference to Figure 3, the cartridge 12 abuts a pump driver 70 (See Figure 3) comprising a platen 72 having a recessed surface 74 therein and a fluid port 76. In use, the platen 70 is kinematically located against the cartridge 12 to sealingly engage with the cartridge 12 such that the recessed surface 74 and the flexible diaphragm 16 define a drive chamber 86. A sensor (not shown) detects whether the cartridge 12 is located correctly and generates an alarm signal if the cartridge 12 is incorrectly located. The cartridge 12 is held against the platen 70 by a door (not shown) and a sensor (not shown) detects whether the door is open or closed.

The fluid port 76 is connectable with a source of positive fluid pressure 78 and a negative source of fluid pressure 80 via a modulated valve 82, controlled by the controller 84 to allow fluid to flow into or out of the drive chamber 86.

The modulated valve 82 is a proportional valve having a variable sized orifice therein, the valve being controllable to change the size of the orifice, thereby controlling the flow of fluid therethrough.

The sources of positive and negative fluid pressure 78, 80 include a pressure pump and a vacuum pump respectively. When the modulated valve 82 is operated to allow fluid to flow into the drive chamber 86 from the source of positive fluid pressure 78, the flexible diaphragm 16a moves towards the recessed surface 14 and any blood that is in the pump chamber 18, 22 is pumped out of the common inlet and outlet 20. When the modulated valve 82 is operated to allow fluid to flow out of the drive chamber 86 to the source of negative fluid pressure 80, the flexible diaphragm 16b is moved away from the recessed surface 14 towards surface 74 and blood is drawn into the pump chamber 18, 22 from the common inlet and outlet 20.

In order to pump blood through the pump chambers 18, 22, the common inlet and outlet 20 of each pump 18, 22 has an inlet valve 30, 32 and an outlet valve 36, 38 associated therewith. In use, when the modulated valve 82 is operated to allow fluid into the drive chamber 86 from the source of positive fluid pressure 78, the inlet valve 30, 32 of the pump chamber 18, 22 is closed and the outlet valve 36, 38 of the pump chamber 18, 22 is open so that the blood within the pump chamber 18, 22 exits the common inlet and outlet 20 via the outlet valve 36, 38 of the pump chamber 18, 22.

When the modulated valve 82 is operated to allow fluid to flow out of the drive chamber 86 to the source of negative fluid pressure 80, the inlet valve 30, 32 of the pump chamber 18, 22 is opened and the outlet valve 36, 38 of the pump chamber 18, 22 is closed such that blood is drawn into the pump chamber 18, 22 through the common inlet and outlet 20 via the open inlet valve 30, 32 of the pump chamber 18, 22.

The inlet valves 30, 32 and outlet valves 36, 38 of the pump chambers 18, 22 are, in use, configured to operate to minimise pressure spikes in the patients blood.

When changing from filling to emptying the pump chamber 18, 22, the inlet valve 30, 32 of the pump chamber 18, 22 is closed and the outlet valve 36, 38 of the pump chamber 18, 22 is opened before flow of blood commences from the pump chamber. Opening the outlet valve 36, 38 of the pump chamber 18, 22 before flow of blood commences from the pump chamber 18, 22 ensures that there is no resistance against the flow of blood out of the common inlet and outlet 20. The outlet valve 36, 38 of the pump chamber 18, 22 is not opened instantaneously. Opening the outlet valve 36, 38 of the pump chamber 18, 22 at the same time as flow of blood commences from the pump chamber 18, 22 would create a positive pressure spike within the blood and cause rupturing of red blood cells.

When changing from emptying to filling the pump chamber 18, 22, the outlet valve 36, 38 of the pump chamber 18, 22 is closed and the inlet valve 30, 32 of the pump chamber 18, 22 is opened before flow of blood commences to the pump chamber 18, 22. Opening the inlet valve 30, 32 of the pump chamber 18, 22 before flow of blood commences to the pump chamber 18, 22 ensures that there is no resistance against the flow of blood into the common inlet and outlet 20 of the pump chamber 18, 22. The inlet valve 30, 32 of the pump chamber 18, 22 is not opened instantaneously. Opening the inlet valve 30, 32 of the pump chamber 18, 22 at the same time as flow of blood commences into the pump chamber 18, 22 would create a negative pressure spike within the blood and cause rupturing of red blood cells.

The inlet valves 30, 32 and the outlet valves 36, 38 of the pump chambers 18, 22 may be operated such that when the flexible diaphragm 16 of a pump chamber 18, 22 is at one extremity of its travel, either adjacent the concave recess 14 or adjacent the recessed surface 72, the valve of the pump chamber 18, 22, that is opening opens before the valve of the pump chamber 18, 22 that is closing closes, .i.e. both valves of the pump chamber are momentarily open.

For example, when positive pressure is applied to the flexible diaphragm 16 it travels in the direction towards the concave recess 14, displacing blood through the common inlet and outlet 20 via the open outlet valve 36, 38 of the pump chamber 18, 22.

Once the flexible diaphragm 16a has reached the concave recess 14, the inlet valve 30, 32 of the pump chamber 18, 22 is first opened, the outlet valve 36, 38 of the pump chamber 18, 22 is then closed and then the modulated valve 82 is operated to allow fluid to flow out of the drive chamber 86 such that the flexible diaphragm 16 starts to move in the direction away from the concave recess 14 and towards the recessed surface 72.

In a similar manner, when the diaphragm 16b reaches the extremity of its travel adjacent the recessed surface 72, the outlet valve 36, 38 of the pump chamber 18, 22 is first opened, the inlet valve 30, 32 of the pump chamber 18, 22 is then closed, and the modulated valve 82 is then operated to allow fluid to flow into the drive chamber 86 such that the flexible diaphragm 16b starts to move in the direction away from the recessed surface 72 and towards the concave recess 14.

Although the blood pump(s) is/are described with reference to a pump chamber 18, 22 having a single common inlet and outlet 20, each pump chamber 18, 22 could also be provided with two inlet ports and two outlet ports while having the same effect in minimising stasis within the patients blood. Each pump chamber 18, 22 could also be provided with more than two inlet ports and a corresponding number of outlet ports.

In another embodiment, the blood pump is a disposable blood pump comprising a disposable pump cartridge.

The embodiments of the invention, described with reference to the figures, are examples only and do not exclude variations therefrom from the scope of the invention as defined by the claims.

## Claims

1. A blood pump (10) comprising:
a cartridge (12), the cartridge comprising a first recess therein, said first recess having a surface (14), and a flexible diaphragm (16) closing said first recess, the first recess and the flexible diaphragm (16) defining a first pump chamber (18), said first pump chamber (18) having an inlet and an outlet (20) wherein the flexible diaphragm (16) of the first pump chamber (18) is movable between a first position, separated in use from the surface (14) of the first recess, wherein in said first position said first pump chamber (18) has a maximum volume, and a second position, substantially adjacent to the surface (14) of the first recess, wherein in said second position said first pump chamber (18) has a minimum volume;
a pump driver (70) arranged to interface with the cartridge (12), said pump driver (70) operable to move the flexible diaphragm (16) of the first pump chamber (18) in a first direction into said first recess to, in use, pump blood from the chamber (18) and to move the flexible diaphragm (16) of the first pump chamber (18) in a second direction away from the first recess to, in use, draw blood into said first pump chamber (18),
the cartridge (12) further comprising one or more sensor cavities (26, 40, 46) defined by respective recesses in the cartridge (12), the, or each, recess being closed by a flexible diaphragm,
**characterised in that** the, or each sensor cavity (26, 40, 46) is hemispherical, and comprises a surface, said surface defining a flat bottom in a recess.

2. A blood pump (10) according to Claim 1, wherein the cartridge (12) further comprises a second recess therein, said second recess having a surface (14), and a flexible diaphragm (16) closing said second recess, the second recess and the flexible diaphragm (16) defining a second pump chamber (22), said second pump chamber (22) having an inlet and an outlet (20) wherein the flexible diaphragm (16) of the second pump chamber (22) is movable between a first position, separated in use from the surface (14) of the second recess, in which said second pump chamber (22) has a maximum volume, and a second position, substantially adjacent to the surface (14) of the second recess, in which said second pump chamber (22) has a minimum volume.

3. A blood pump (10) according to Claim 1 or 2, wherein the, or each, sensor cavity (26, 40, 46) further comprises an inlet (27, 39, 45) and an outlet (29, 41, 47).

4. A blood pump (10) according to Claim 3, wherein the inlet (27, 39, 45) and outlet (29, 41, 47) of the, or each, sensor cavity (26, 40, 46) is tangential.

5. A blood pump (10) according to claim 3, wherein the inlet (27, 39, 45) is offset from the outlet (29, 41, 47).

6. A blood pump (10) according to claim 3, wherein one of the inlet (27, 39, 45) or the outlet (29, 41, 47) are offset radially with respect to the sensor cavity (26, 40, 46).

7. A blood pump (10) according to any of Claims 2 to 6, wherein the first pump chamber (18) has a common inlet and outlet (20) and the second pump chamber (22) has a common inlet and outlet (20), and/or wherein the blood pump (10) further comprises an arterial blood inlet (24), a dialyser blood outlet (42), a dialyser blood inlet (44) and a venous blood outlet (48) and/or wherein the blood pump (10) comprises three sensor cavities (26, 40, 46).

8. A blood pump (10) according to Claim 7, wherein the sensor cavities (26, 40, 46) respectively define an arterial pressure chamber (26), a dialyser pressure chamber (40) and a venous pressure chamber (46).

9. A blood pump (10) according to Claims 7 or 8, wherein the arterial blood inlet (24) is in fluid connection with the first pump chamber (18) and the second pump chamber (22), and/or wherein the dialyser blood outlet (42) is in fluid connection with the first pump chamber (18) and the second pump chamber (22).

10. A blood pump (10) according to Claim 9, wherein the blood pump (10) further comprises an inlet channel (28) between the arterial blood inlet (24) and the first pump chamber (18) and the second pump chamber (22), and preferably wherein the blood pump (10) further comprises an outlet channel (34) between the dialyser blood outlet (42) and the first pump chamber (18) and the second pump chamber (22).

11. A blood pump (10) according to Claim 10, wherein the blood pump (10) further comprises an inlet valve (30) to the first pump chamber (18) and an inlet valve (32) to the second pump chamber (22), each inlet valve (30, 32) being disposed in the inlet channel (28), preferably wherein the blood pump (10) further comprises an outlet valve (36) from the first pump chamber (18) and an outlet valve (38) from the second pump chamber (22), each outlet valve (36, 38) being disposed in the outlet channel (34).

12. A blood pump (10) according to any of Claims 9 to 11, wherein the arterial pressure chamber (26) is provided downstream of the arterial blood inlet (24) and upstream of the first (18) and second (22) pump chambers, and/or wherein dialyser pressure chamber (40) is provided downstream of the first (18) and second (22) pump chambers and upstream of the dialyser blood outlet (42), and/or wherein the venous pressure chamber (46) is provided downstream of the dialyser blood inlet (44) and upstream of the venous blood outlet (48).

13. A blood pump (10) according to any preceding claim, wherein the, or each, pump chamber (18, 22), sensor cavity (26, 40, 46), valve (30, 32, 36, 38) and channel (28, 34) is provided on a common datum face, and/or wherein the blood pump (10) is disposable, and/or wherein the flexible diaphragm (16) of the, or each, pump chamber and the, or each, sensor cavity (26, 40, 46) is formed from a single sheet of material, and/or wherein the sheet of material defining respective diaphragms is vacuum attached to the cartridge (12) in a region around the, or each, sensor cavity (26, 40, 46).

14. A blood pump (10) according to any preceding claim, the blood pump (10) further comprising a bubble trap (60), preferably wherein the bubble trap (60) is downstream of the, or each, pump chamber (18, 22) and/or wherein the blood pump (10) further comprises a thrombus trap (59), preferably wherein the thrombus trap (59) is provided within a sensor cavity (26, 40, 46).

15. A blood pump (10) according to claim 1, wherein the, or each pump chamber (18, 22) and the, or each, sensor cavity (26, 40, 46) are provided on a common datum face, preferably wherein the cartridge (12) further comprises an inlet valve (30, 32) associated with the, or each, pump chamber (18, 22) and an outlet valve (36, 38) associated with the, or each, pump chamber (18, 22), said inlet valve (30, 32) and said outlet valve (36, 38) being provided on the common datum face of the cartridge (12), and/or wherein the cartridge (12) further comprises an inlet channel (28) to the, or each pump chamber (18, 22) and an outlet channel (34) from the, or each, pump chamber (18, 22), said inlet channel (28) and said outlet channel (34) being provided on the common datum face of the cartridge (12).

## Patentansprüche

1. Blutpumpe (10), umfassend:
eine Kartusche (12), wobei die Kartusche eine erste Vertiefung darin und eine flexible Membran (16) umfasst, wobei die erste Vertiefung eine Oberfläche (14) aufweist und die flexible Membran (16) die erste Vertiefung verschließt, wobei die erste Vertiefung und die flexible Membran (16) eine erste Pumpenkammer (18) definieren, wobei die erste Pumpenkammer (18) einen Einlass und einen Auslass (20) aufweist, wobei die flexible Membran (16) der ersten Pumpenkammer (18) zwischen einer im Gebrauch von der Oberfläche der ersten Vertiefung abgetrennten ersten Position und einer im Wesentlichen zu der Oberfläche (14) der ersten Vertiefung benachbarten zweiten Position bewegbar ist, wobei in der ersten Position die erste Pumpenkammer (18) ein maximales Volumen und in der zweiten Position die erste Pumpenkammer (18) ein minimales Volumen aufweist;
einen Pumpenantrieb (70), der eingerichtet ist, mit der Kartusche (12) über eine Schnittstelle verbunden zu sein, wobei der Pumpenantrieb (70) fähig ist, die flexible Membran (16) der ersten Pumpenkammer (18) in einer ersten Richtung in die erste Vertiefung hinein zu bewegen, um im Gebrauch Blut aus der Kammer (18) zu pumpen und die flexible Membran (16) der ersten Pumpenkammer (18) in einer zweiten Richtung weg von der ersten Vertiefung zu bewegen, um im Gebrauch Blut in die erste Pumpenkammer (18) zu saugen,
wobei die Kartusche (12) ferner eine oder mehrere durch jeweilige Vertiefungen in der Kartusche (12) definierte Sensorhohlräume (26, 40, 46) aufweist, wobei die oder jede Vertiefung durch eine flexible Membrane geschlossen ist,
**dadurch gekennzeichnet, dass** der oder jeder Sensorhohlraum (26, 40, 46) halbkugelförmig ist und eine Oberfläche aufweist, wobei die Oberfläche einen flachen Boden in einer Vertiefung definiert.

2. Blutpumpe (10) nach Anspruch 1, wobei die Kartusche (12) ferner eine eine Oberfläche (14) aufweisende zweite Vertiefung darin und eine die zweite Vertiefung verschließende flexible Membran (16) umfasst, wobei die zweite Vertiefung und die flexible Membran (16) eine zweite Pumpenkammer (22) definieren, wobei die zweite Pumpenkammer (22) einen Einlass und einen Auslass (20) aufweist, wobei die flexible Membran (16) der zweiten Pumpenkammer (22) zwischen einer im Gebrauch von der Oberfläche (14) der zweiten Vertiefung abgetrennten ersten Position, in der die zweite Pumpenkammer (22) ein maximales Volumen aufweist, und einer im Wesentlichen zu der Oberfläche (14) der zweiten Vertiefung benachbarten zweiten Position, in der die zweite Pumpenkammer (22) ein minimales Volumen aufweist, bewegbar ist.

3. Blutpumpe (10) nach Anspruch 1 oder 2, wobei der oder jeder Sensorhohlraum (26, 40, 46) ferner einen Einlass (27, 39, 45) und einen Auslass (29, 41, 47) umfasst.

4. Blutpumpe (10) nach Anspruch 3, wobei der Einlass (27, 39, 45) und der Auslass (29, 41, 47) des oder jedes Sensorhohlraums (26, 40, 46) tangential ist.

5. Blutpumpe (10) nach Anspruch 3, wobei der Einlass (27, 39, 45) gegenüber dem Auslass (29, 41, 47) versetzt ist.

6. Blutpumpe (10) nach Anspruch 3, wobei einer von dem Einlass (27, 39, 45) oder dem Auslass (29, 41, 47) radial in Bezug auf den Sensorhohlraum (26, 40, 46) versetzt ist.

7. Blutpumpe (10) nach einem der Ansprüche 2 bis 6, wobei die erste Pumpenkammer (18) einen gemeinsamen Einlass und Auslass (20) aufweist und die zweite Pumpenkammer (22) einen gemeinsamen Einlass und Auslass (20) aufweist und/oder wobei die Blutpumpe (10) ferner einen Arterienbluteinlass (24), einen Dialysatorblutauslass (42), einen Dialysatorbluteinlass (44) und einen Venenblutauslass (48) umfasst und/oder wobei die Blutpumpe (10) drei Sensorhohlräume (26, 40, 46) umfasst.

8. Blutpumpe (10) nach Anspruch 7, wobei die Sensorhohlräume (26, 40, 46) jeweils eine Arteriendruckkammer (26), eine Dialysatordruckkammer (40) und eine Venendruckkammer (46) definieren.

9. Blutpumpe (10) nach einem der Ansprüche 7 bis 8, wobei der Arterienbluteinlass (24) in Fluidverbindung mit der ersten Pumpenkammer (18) und der zweiten Pumpenkammer (22) steht und/oder wobei der Dialysatorblutauslass (42) in Fluidverbindung mit der ersten Pumpenkammer (18) und der zweiten Pumpenkammer (22) steht.

10. Blutpumpe (10) nach Anspruch 9, wobei die Blutpumpe (10) ferner einen Einlasskanal (28) zwischen dem Arterienbluteinlass (24) und der ersten Pumpenkammer (18) und der zweiten Pumpenkammer (22) umfasst und vorzugsweise wobei die Blutpumpe (10) ferner einen Auslasskanal (34) zwischen dem Dialysatorblutauslass (42) und der ersten Pumpenkammer (18) und der zweiten Pumpenkammer (22) umfasst.

11. Blutpumpe (10) nach Anspruch 10, wobei die Blutpumpe (10) ferner ein Einlassventil (30) zu der ersten Pumpenkammer (18) und ein Einlassventil (32) zu der zweiten Pumpenkammer (22) umfasst, wobei jedes Einlassventil (30, 32) in dem Einlasskanal (28) angeordnet ist, vorzugsweise wobei die Blutpumpe (10) ferner ein Auslassventil (36) von der ersten Pumpenkammer (18) und ein Auslassventil (38) von der zweiten Pumpenkammer (22) umfasst, wobei jedes Auslassventil (36, 38) in dem Auslasskanal (34) angeordnet ist.

12. Blutpumpe (10) nach einem der Ansprüche 9 bis 11, wobei die Arteriendruckkammer (26) stromabwärts des Arterienbluteinlasses (24) und stromaufwärts von der ersten (18) und der zweiten (22) Pumpenkammer vorgesehen ist und/oder wobei Dialysatordruckkammer (40) stromabwärts von der ersten (18) und der zweiten (22) Pumpenkammer und stromaufwärts des Dialysatorblutauslasses (42) vorgesehen ist und/oder wobei die Venendruckkammer (46) stromabwärts von dem Dialysatorbluteinlass (44) und stromaufwärts von dem Venenblutauslass (48) vorgesehen ist.

13. Blutpumpe (10) nach einem der vorhergehenden Ansprüche, wobei die (der/das) oder jede(r/s) Pumpkammer (18, 22), Sensorhohlraum (26, 40, 46), Ventil (30, 32, 36, 38) und Kanal (28, 34) auf einer gemeinsamen Bezugsfläche vorgesehen sind und/oder wobei die Blutpumpe (10) wegwerfbar ist und/oder wobei die flexible Membran (16) der oder jeder Pumpenkammer und der oder jeder Sensorhohlraum (26, 40, 46) aus einem einzigen Materialblatt gebildet ist und/oder wobei das Materialblatt, das jeweilige Membranen definiert, in einem Bereich um den oder jeden Sensorhohlraum (26, 40, 46) herum mittels Vakuum an der Kartusche (12) angebracht ist.

14. Blutpumpe (10) nach einem der vorhergehenden Ansprüche, wobei die Blutpumpe (10) ferner eine Blasenfalle (60) aufweist, wobei vorzugsweise sich die Blasenfalle (60) stromabwärts von der oder jeder Pumpenkammer (18, 22) befindet und/oder wobei die Blutpumpe (10) ferner eine Thrombusfalle (59) umfasst, wobei vorzugsweise die Thrombusfalle (59) in einem Sensorhohlraum (26, 40 46) vorgesehen ist.

15. Blutpumpe (10) nach Anspruch 1, wobei die oder jede Pumpkammer (18, 22), und der oder jeder Sensorhohlraum (26, 40, 46) auf einer gemeinsamen Bezugsfläche vorgesehen sind, vorzugsweise wobei die Kartusche (12) ferner ein der oder jeder Pumpenkammer (18, 22) zugeordnetes Einlassventil (30, 32) und ein der oder jeder Pumpenkammer (18, 22) zugeordnetes Auslassventil (36, 38) umfasst, wobei das Einlassventil (30, 32) und das Auslassventil (36, 38) an der gemeinsamen Bezugsfläche der Kartusche (12) vorgesehen sind und/oder wobei die Kartusche (12) ferner einen Einlasskanal (28) zu der oder jeder Pumpenkammer (18, 22) und einen Auslasskanal (34) von der oder jeder Pumpenkammer (18, 22) umfasst, wobei der Einlasskanal (28) und der Auslasskanal (34) auf der gemeinsamen Bezugsfläche der Kartusche (12) vorgesehen sind.

## Revendications

1. Pompe à sang (10) comprenant :
une cartouche (12), la cartouche comprenant un premier évidement dans cette dernière, ledit premier évidement ayant une surface (14), et un diaphragme flexible (16) fermant ledit premier évidement, le premier évidement et le diaphragme flexible (16) définissant une première chambre de pompe (18), ladite première chambre de pompe (18) ayant une entrée et une sortie (20), dans laquelle le diaphragme flexible (16) de la première chambre de pompe (18) est mobile entre une première position séparée, à l'usage, de la surface (14) du premier évidement, dans laquelle, dans ladite première position, ladite première chambre de pompe (18) a un volume maximum, et une seconde position sensiblement adjacente à la surface (14) du premier évidement, dans laquelle dans ladite seconde position, ladite première chambre de pompe (18) a un volume minimum ;
un dispositif d'entraînement de pompe (70) agencé pour s'interfacer avec la cartouche (12), ledit dispositif d'entraînement de pompe (70) étant opérationnel pour déplacer le diaphragme flexible (16) de la première chambre de pompe (18) dans une première direction dans ledit premier évidement pour, à l'usage, pomper le sang de la chambre (18) et pour déplacer le diaphragme flexible (16) de la première chambre de pompe (18) dans une seconde direction à distance du premier évidement pour, à l'usage, aspirer le sang dans ladite première chambre de pompe (18),
la cartouche (12) comprenant en outre une ou plusieurs cavités de capteur (26, 40, 46) définies par des évidements respectifs dans la cartouche (12), le ou chaque évidement étant fermé par un diaphragme flexible,
**caractérisée en ce que** la ou chaque cavité de capteur (26, 40, 46) est hémisphérique, et comprend une surface, ladite surface définissant un fond plat dans un évidement.

2. Pompe à sang (10) selon la revendication 1, dans laquelle la cartouche (12) comprend en outre un second évidement dans cette dernière, ledit second évidement ayant une surface (14) et un diaphragme flexible (16) fermant ledit second évidement, le second évidement et le diaphragme flexible (16) définissant une seconde chambre de pompe (22), ladite seconde chambre de pompe (22) ayant une entrée et une sortie (20) dans laquelle le diaphragme flexible (16) de la seconde chambre de pompe (22) est mobile entre une première position séparée, à l'usage, de la surface (14) du second évidement, dans laquelle ladite seconde chambre de pompe (22) a un volume maximum, et une seconde position sensiblement adjacente à la surface (14) du second évidement, dans laquelle ladite seconde chambre de pompe (22) a un volume minimum.

3. Pompe à sang (10) selon la revendication 1 ou 2, dans laquelle la ou chaque cavité de capteur (26, 40, 46) comprend en outre une entrée (27, 39, 45) et une sortie (29, 41, 47).

4. Pompe à sang (10) selon la revendication 3, dans laquelle l'entrée (27, 39, 45) et la sortie (29, 41, 47) de la ou de chaque cavité de capteur (26, 40, 46) est tangentielle.

5. Pompe à sang (10) selon la revendication 3, dans laquelle l'entrée (27, 39, 45) est décalée de la sortie (29, 41, 47).

6. Pompe à sang (10) selon la revendication 3, dans laquelle l'une parmi l'entrée (27, 39, 45) ou la sortie (29, 41, 47) est radialement décalée par rapport à la cavité de capteur (26, 40, 46).

7. Pompe à sang (10) selon l'une quelconque des revendications 2 à 6, dans laquelle la première chambre de pompe (18) a une entrée et une sortie (20) communes et la seconde chambre de pompe (22) a une entrée et une sortie (20) communes, et/ou dans laquelle la pompe à sang (10) comprend en outre une entrée de sang artériel (24), une sortie de sang de dialyseur (42), une entrée de sang de dialyseur (44) et une sortie de sang veineux (48) et/ou dans laquelle la pompe à sang (10) comprend trois cavités de capteur (26, 40, 46).

8. Pompe à sang (10) selon la revendication 7, dans laquelle les cavités de capteur (26, 40, 46) définissent respectivement une chambre de pression artérielle (26), une chambre de pression de dialyseur (40) et une chambre de pression veineuse (46).

9. Pompe à sang (10) selon les revendications 7 ou 8, dans laquelle l'entrée de sang artériel (24) est en raccordement de fluide avec la première chambre de pompe (18) et la seconde chambre de pompe (22), et/ou dans laquelle la sortie de sang de dialyseur (42) est en raccordement de fluide avec la première chambre de pompe (18) et la seconde chambre de pompe (22).

10. Pompe à sang (10) selon la revendication 9, dans laquelle la pompe à sang (10) comprend en outre un canal d'entrée (28) entre l'entrée de sang artériel (24) et la première chambre de pompe (18) et la seconde chambre de pompe (22), et de préférence dans laquelle la pompe à sang (10) comprend en outre un canal de sortie (34) entre la sortie de sang de dialyseur (42) et la première chambre de pompe (18) et la seconde chambre de pompe (22).

11. Pompe à sang (10) selon la revendication 10, dans laquelle la pompe à sang (10) comprend en outre une valve d'entrée (30) dans la première chambre de pompe (18) et une valve d'entrée (32) dans la seconde chambre de pompe (22), chaque valve d'entrée (30, 32) étant disposée dans le canal d'entrée (28), de préférence dans laquelle la pompe à sang (10) comprend en outre une valve de sortie (36) de la première chambre de pompe (18) et une valve de sortie (38) de la seconde chambre de pompe (22), chaque valve de sortie (36, 38) étant disposée dans le canal de sortie (34).

12. Pompe à sang (10) selon l'une quelconque des revendications 9 à 11, dans laquelle la chambre de pression artérielle (26) est prévue en aval de l'entrée de sang artériel (24) et en amont des première (18) et seconde (22) chambres de pompe, et/ou dans laquelle la chambre de pression de dialyseur (40) est prévue en aval des première (18) et seconde (22) chambres de pompe et en amont de la sortie de sang de dialyseur (42), et/ou dans laquelle la chambre de pression veineuse (46) est prévue en aval de l'entrée de sang de dialyseur (44) et en amont de la sortie de sang veineux (48).

13. Pompe à sang (10) selon l'une quelconque des revendications précédentes, dans laquelle la (le) ou chacune (chacun) parmi la chambre de pompe (18, 22), la cavité de capteur (26, 40, 46), la valve (30, 32, 36, 38) et le canal (28, 34) est prévu(e) sur une face de données commune, et/ou dans laquelle la pompe à sang (10) est jetable, et/ou dans laquelle le diaphragme flexible (16) de la ou de chaque chambre de pompe et de la ou de chaque cavité de capteur (26, 40, 46) est formé à partir d'une seule feuille de matériau, et/ou dans laquelle la feuille de matériau définissant des diaphragmes respectifs est fixée sous vide sur la cartouche (12) dans une région autour de la ou de chaque cavité de capteur (26, 40, 46).

14. Pompe à sang (10) selon l'une quelconque des revendications précédentes, la pompe à sang (10) comprenant en outre un piège à bulles (60), de préférence dans laquelle le piège à bulles (60) est en aval de la ou de chaque chambre de pompe (18, 22) et/ou dans laquelle la pompe à sang (10) comprenant en outre un piège à thrombus (59), de préférence dans laquelle le piège à thrombus (59) est prévu à l'intérieur d'une cavité de capteur (26, 40, 46).

15. Pompe à sang (10) selon la revendication 1, dans laquelle la ou chaque chambre de pompe (18, 22) et la ou chaque cavité de capteur (26, 40, 46) sont prévues sur une face de données commune, de préférence dans laquelle la cartouche (12) comprend en outre une valve d'entrée (30, 32) associée avec la ou chaque chambre de pompe (18, 22) et une valve de sortie (36, 38) associée avec la ou chaque chambre de pompe (18, 22), ladite valve d'entrée (30, 32) et ladite valve de sortie (36, 38) étant prévue sur la face de données commune de la cartouche (12), et/ou dans laquelle la cartouche (12) comprenant en outre un canal d'entrée (28) dans la ou chaque chambre de pompe (18, 22) et un canal de sortie (34) de la ou chaque chambre de pompe (18, 22), ledit canal d'entrée (28) et ledit canal de sortie (34) étant prévus sur la face de données commune de la cartouche (12).
